# FASCICULE DE BREVET EUROPEEN

(11) **EP 0 909 756 B1**
(45) Date de publication et mention de la délivrance du brevet: **25.07.2001**
(21) Numéro de dépôt: 98402405.9
(22) Date de dépôt: 30.09.1998
(51) Int. Cl.: C07C 327/06

(54) **Stabilisation de l'acide thioacétique**
Stabilisierung von Thioessigsäure
Stabilization of thioacetic acid

(30) Priorité: 15.10.1997 FR 9712898
(43) Date de publication de la demande: 21.04.1999
(73) Titulaire: Atofina, 92800 Puteaux (FR)
(72) Inventeur: Labat, Yves, 64000 Pau (FR); Monguillon, Bernard, 64320 Bizanos (FR)
(74) Mandataire: Leboulenger, Jean

(56) Documents cités:
- PATENT ABSTRACTS OF JAPAN vol. 009, no. 306, 3 décembre 1985 & JP 60 142953 A (TOUYOU KASEI KOGYO KK) & JP 01 345605 C

## Description

La présente invention concerne le domaine des acides thiocarboxyliques et a plus particulièrement pour objet la stabilisation de l'acide thioacétique.

Cet acide de formule CH₃-COSH, fabriqué par réaction de l'hydrogène sulfuré avec l'anhydride acétique (brevet FR 1 417 154), est actuellement utilisé comme intermédiaire de synthèse de produits pharmaceutiques tels que le captopryl (agent antihypertensif).

Il est connu que l'acide thioacétique tend, par hydrolyse, à former divers composés soufrés ou oxygénés qui vont faire baisser le titre du produit commercial en acide thioacétique. Cette instabilité de l'acide thioacétique est notamment due à la formation d'hydrogène sulfuré et d'acide acétique selon la réaction : 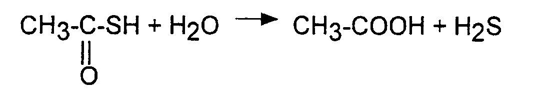 catalysée par des espèces acides (Tetrahedron, 1965, vol. 21, p. 835). Il se forme également du sulfure de diacétyle (CH₃CO-S-COCH₃) et du disulfure de diacétyle (CH₃CO-S-S-COCH₃).

Pour stabiliser l'acide thioacétique, il a été proposé dans le brevet JP 1 345 605 de lui ajouter un acide minéral phosphoré ou un acide organique fort (pKa < 3,3). A part l'acide oxalique, les autres composés cités (acide phosphoreux, acide citrique, acide méthanesulfonique) n'ont pas une action stabilisatrice suffisante pour une bonne conservation de l'acide thioglycolique au stockage.

Il a maintenant été trouvé, de façon surprenante, que les acides chloroacétiques permettent de bien stabiliser à 50°C l'acide thioacétique et ceci pendant une période de 1 à 2 mois nécessaire au transport de ce produit sur de longues distances.

On a également constaté que, dans la série des acides chloroacétiques comprenant l'acide monochloroacétique (pKa = 2,9), l'acide dichloroacétique (pKa = 1,3) et l'acide trichloroacétique (pKa = 0,7), l'acide dichloroacétique présente l'action stabilisante la plus efficace.

L'invention a donc pour premier objet un procédé de stabilisation de l'acide thioacétique, caractérisé en ce qu'on lui ajoute une quantité suffisante d'un acide chloroacétique, de préférence l'acide dichloroacétique.

L'invention a également pour objet une composition constituée essentiellement d'acide thioacétique et d'une quantité mineure d'un acide chloroacétique, de préférence l'acide dichloroacétique.

La quantité d'acide chloroacétique stabilisant peut aller de 50 à 5000 ppm, mais est de préférence comprise entre 100 et 2000 ppm (0,01 à 0,2 % en poids).

Pour obtenir l'action stabilisante optimale, il est avantageux d'ajouter l'acide chloroacétique stabilisant à un acide thioacétique fraîchement distillé et exempt, autant que possible, d'eau (eau < 0,5 %). D'autre part, pour éviter l'action oxydante de l'air, il est souhaitable d'isoler le produit stocké sous azote.

Les exemples suivants illustrent l'invention sans la limiter.

### EXEMPLE 1 comparatif

De l'acide thioacétique fraîchement distillé (pureté = 99,27 %) a été introduit dans plusieurs ampoules en verre que l'on a scellées et maintenues dans une étuve à 50°C.

Périodiquement, les ampoules ont été refroidies et ouvertes pour doser par chromatographie gazeuse le titre en acide thioacétique. Les résultats qui figurent dans le tableau suivant montrent qu'après 100 jours de stockage la pureté du produit est tombée à 63 %.

### EXEMPLE 2 comparatif

On a opéré comme à l'exemple 1, mais en ajoutant à l'acide thioacétique 3000 ppm d'acide oxalique. Après 100 jours de stockage, la perte de pureté est d'environ 10 %.

### EXEMPLES 3 ET 4

On a opéré comme à l'exemple 1, mais en ajoutant à l'acide thioacétique 3000 ppm d'acide dichloroacétique (exemple 3) ou seulement 1000 ppm de ce même acide (exemple 4).

Les résultats du tableau suivant montrent que l'acide dichloroacétique est bien plus efficace que l'acide oxalique puisqu'après 100 jours de stockage, le titre en acide thioacétique n'a chuté que 2-3 % et cela même avec une dose trois fois moindre (exemple 4).

| **DUREE DE STOCKAGE A 50°C (jours)** | **PURETE (%) DE L'ACIDE THIOACETIQUE** | | | |
|---|---|---|---|---|
| | **Exemple 1 comparatif** | **Exemple 2 comparatif** | **Exemple 3** | **Exemple 4** |
| 0 | 99,27 | 99,27 | 99,27 | 99,27 |
| 20 | 93,60 | 98,80 | 98,85 | 99,0 |
| 50 | 91,80 | 97,80 | 98,50 | 98,96 |
| 80 | 80 | 94,20 | 97,10 | 98,40 |
| 100 | 63 | 88,90 | 96,40 | 96,80 |

## Revendications

1. Procédé de stabilisation de l'acide thioacétique, caractérisé en ce qu'on lui ajoute une quantité suffisante d'un acide chloroacétique.

2. Procédé selon la revendication 1 dans lequel le stabilisant est l'acide dichloroacétique.

3. Procédé selon la revendication 1 ou 2 dans lequel la quantité de stabilisant va de 50 à 5000 ppm et, de préférence, est comprise entre 100 et 2000 ppm.

4. Procédé selon l'une des revendications 1 à 3 dans lequel le stabilisant est ajouté à un acide thioacétique fraîchement distillé, de préférence à un acide thioacétique ayant une teneur en eau inférieure à 0,5 %.

5. Procédé selon l'une des revendications 1 à 4 dans lequel le produit stabilisé est stocké à l'abri de l'air.

6. Composition constituée essentiellement d'acide thioacétique avec 50 à 5000 ppm d'un acide chloroacétique.

7. Composition selon la revendication 6 contenant 100 à 2000 ppm d'acide chloroacétique.

8. Composition selon la revendication 6 ou 7 dans laquelle l'acide chloroacétique est l'acide dichloroacétique.

## Claims

1. Process for stabilizing thioacetic acid, characterized in that a sufficient amount of a chloroacetic acid is added thereto.

2. Process according to Claim 1, in which the stabilizer is dichloroacetic acid.

3. Process according to Claim 1 or 2, in which the amount of stabilizer ranges from 50 to 5000 ppm and is preferably between 100 and 2000 ppm.

4. Process according to one of Claims 1 to 3, in which the stabilizer is added to a freshly distilled thioacetic acid, preferably to a thioacetic acid having a water content of less than 0.5%.

5. Process according to one of Claims 1 to 4, in which the stabilized product is stored in the absence of air.

6. Composition consisting essentially of thioacetic acid with 50 to 5000 ppm of a chloroacetic acid.

7. Composition according to Claim 6, containing 100 to 2000 ppm of chloroacetic acid.

8. Composition according to Claim 6 or 7, in which the chloroacetic acid is dichloroacetic acid.

## Patentansprüche

1. Verfahren zur Stabilisierung von Thioessigsäure, dadurch gekennzeichnet, daß man ihr eine ausreichende Menge einer Chloressigsäure zugibt.

2. Verfahren nach Anspruch 1, in dem der Stabilisator Dichloressigsäure ist.

3. Verfahren nach Anspruch 1 oder 2, in dem die Menge des Stabilisators von 50 bis 5.000 ppm reicht und vorzugsweise zwischen 100 und 2.000 ppm liegt.

4. Verfahren nach einem der Ansprüche 1 bis 3, in dem der Stabilisator einer kurz zuvor destillierten Thioessigsäure, vorzugsweise einer Thioessigsäure mit einem Wassergehalt kleiner 0,5 %, zugegeben wird.

5. Verfahren nach einem der Ansprüche 1 bis 4, in dem das stabilisierte Produkt luftdicht verschlossen gespeichert wird.

6. Zusammensetzung, die im wesentlichen aus Thioessigsäure mit 50 bis 5.000 ppm einer Chloressigsäure besteht.

7. Zusammensetzung nach Anspruch 6, die 100 bis 2.000 ppm Chloressigsäure enthält.

8. Zusammensetzung nach Anspruch 6 oder 7, in der die Chloressigsäure Dichloressigsäure ist.
